# EUROPÄISCHE PATENTSCHRIFT

(11) **EP 2 915 492 B1**
(45) Veröffentlichungstag und Bekanntmachung des Hinweises auf die Patenterteilung: **18.10.2017**
(21) Anmeldenummer: 15157580.0
(22) Anmeldetag: 04.03.2015
(51) Int. Cl.: A61B 17/04, A61B 17/062, A61B 17/29

(54) **INSTRUMENT ZUM CHIRURGISCHEN NÄHEN BEI DER MINIMAL-INVASIVEN CHIRURGIE UND NADELHALTERKUPPLUNG FÜR EIN DERARTIGES INSTRUMENT**
INSTRUMENT FOR PERFORMING SURGICAL SUTURES IN MINIMALLY INVASIVE SURGERY AND NEEDLE HOLDER COUPLING FOR SUCH AN INSTRUMENT
INSTRUMENT DESTINÉ À LA SUTURE CHIRURGICALE LORS DE CHIRURGIE MINI-INVASIVE ET COUPLAGE DE PORTE-AIGUILLE POUR UN TEL INSTRUMENT

(30) Priorität: 04.03.2014 AT 501602014
(43) Veröffentlichungstag der Anmeldung: 09.09.2015
(73) Patentinhaber: AFSMEDICAL GmbH Medizinproduktehandel, 2524 Teesdorf (AT)
(72) Erfinder: Dauser, Bernhard, 1180 Wien (AT)
(74) Vertreter: Sonn & Partner Patentanwälte

(56) Entgegenhaltungen:
- WO-A1-2012/068002
- US-A- 5 984 932
- US-A1- 2002 111 534

## Beschreibung

Die Erfindung betrifft eine Nadelhalterkupplung für ein Instrument zum chirurgischen Nähen bei der minimal-invasiven Chirurgie mit einer über einen Port zu einer Operationsstelle zuführbaren Greifeinrichtung mit einer Zange zum Greifen einer chirurgischen Nadel am distalen Ende und einer Einrichtung zur Betätigung der Zange am proximalen Ende, und mit einem durch den Port einführbaren, die Greifeinrichtung umhüllenden Rohr.

Die Erfindung betrifft weiters ein Instrument zum chirurgischen Nähen bei der minimal-invasiven Chirurgie, insbesondere Einzelport-Chirurgie, mit einer über einen Port zu einer Operationsstelle zuführbaren Greifeinrichtung mit einer Zange zum Greifen einer chirurgischen Nadel am distalen Ende und einer Einrichtung zur Betätigung der Zange am proximalen Ende mit einer oben genannten durch den Port einführbaren Nadelhalterkupplung.

Die vorliegende Erfindung bezieht sich insbesondere auf die Abdominalchirurgie und allenfalls Thoraxchirurgie bei welcher ein Trend zu minimal-invasiven, laparoskopischen Eingriffen mit möglichst wenig Zugängen zum Operationsgebiet zu verzeichnen ist. In jüngster Zeit werden operative Eingriffe nach der sogenannten Einzelport-Chirurgie über einen einzigen Zugang vorgenommen. Der Zugang wird bei der Abdominalchirurgie meist im Bereich des Nabels gelegt, sodass keine sichtbaren Narben von der Operation resultieren. Neben den kosmetischen Vorteilen ist bei der Einzelport-Chirurgie auch die Wundheilung gegenüber konventionellen chirurgischen Methoden verbessert.

Durch wenige Zugänge bei der minimal-invasiven Chirurgie wird die Arbeit des Chirurgen erschwert, da im Wesentlichen sämtliche Instrumente über einen oder zwei Ports an die Operationsstelle herangeführt und entsprechend manövriert werden müssen. Bei der Einzelport-Chirurgie mit nur einem einzigen Port ist die Handhabung für den Chirurgen besonders schwierig. Insbesondere beim chirurgischen Nähen kommt es bei herkömmlichen Instrumenten zu Schwierigkeiten bei der Handhabung. Üblicherweise wird die chirurgische Nadel mit Hilfe von Greifelementen gehalten und nach dem Durchstechen des Gewebes an einen Nadelhalter übergeben und anschließend wieder vom Greifelement übernommen. Zur Unterstützung des chirurgischen Nähens wurden verschiedene Instrumente entwickelt, welche jedoch bei der minimal-invasiven Chirurgie, insbesondere Einzelport-Chirurgie nicht, oder zumindest nicht optimal, verwendet werden können.

Beispielsweise beschreibt die EP 0 908 141 A1 einen Nadelhalter mit zwei Stäben an deren distalen Enden jeweils Greifelemente angeordnet sind, über welche die chirurgische Nadel gehalten werden kann. Über einen komplexen Mechanismus kann die Distanz zwischen den Stäben verändert werden und dadurch das chirurgische Nähen unterstützt werden. Das Gerät mit den voneinander beabstandeten Stäben führt jedoch dazu, dass man Luft durch die Abdichtung am Trokar verlieren würde.

Die US 5 984 932 A beschreibt eine Nadelhalterkupplung für ein Instrument zum chirurgischen Nähen bei der minimal-invasiven Chirurgie, bei der zwei parallel durch den Port hindurchgeführten Greifeinrichtungen zum Greifen einer chriurgischen Nadel vorgesehen sind. Durch die exzentrische Anordnung der Greifeinrichtungen ergibt sich eine für den Chirurgen ungewohnte Bewegung der Nadel. Die Konstruktion bedient sich einer relativ komplexen Mechanik.

Die Aufgabe der vorliegenden Erfindung besteht in der Schaffung einer Nadelhalterkupplung für ein Instruments zum chirurgischen Nähen bei der minimal-invasiven Chirurgie und ein derartiges Instrument, durch welche die Handhabung während des Nähens erleichtert werden kann. Die Konstruktion der Nadelhalterkupplung bzw. des Instruments soll möglichst einfach und robust sein, um eine kostengünstige Herstellung, auch als Einwegprodukt, zu ermöglichen. Nachteile bekannter Vorrichtungen sollen vermieden oder zumindest reduziert werden.

Gelöst wird die erfindungsgemäße Aufgabe durch eine oben erwähnte Nadelhalterkupplung, bei der das Rohr gegenüber der Greifeinrichtung verschiebbar angeordnet ist, und am distalen Ende des Rohres eine gegenüber der Mittellinie des Rohres auslenkbare Vorrichtung zum Halten der chirurgischen Nadel angeordnet ist. Die Konstruktion zeichnet sich durch besondere Einfachheit aus und ermöglicht die gleichzeitige oder sukzessive Einführung der Nadelhalterkupplung und der Greifeinrichtung über den Port zur Operationsstelle. Durch den Abstand zwischen der Vorrichtung zum Halten der chirurgischen Nadel und der Zange der an sich bekannten Greifeinrichtung wird der Nähvorgang unterstützt. Durch entsprechendes Verdrehen der Greifeinrichtung gegenüber dem Rohr der Nadelhalterkupplung, kann die Orientierung der Vorrichtung zum Halten der chirurgischen Nadel gegenüber der Zange der Greifeinrichtung verändert werden. Die erfindungsgemäße Konstruktion ermöglicht eine Verwendung bei konventionell erhältlichen Greifeinrichtungen.

Vorteilhafterweise ist am distalen Ende des Rohres der Nadelhalterkupplung ein elastisch nach außen vorgespanntes Federelement angeordnet, an welchem Federelement die Vorrichtung zum Halten der chirurgischen Nadel befestigt ist. Durch diese Konstruktion wird das Einführen der Nadelhalterkupplung durch den Port ermöglicht bzw. erleichtert, da das nach Außen vorgespannte Federelement während des Hindurchführens durch den Port in Richtung Mittelachse des Rohrs bewegt werden kann. Sobald das Rohr der Nadelhelterkupplung durch den Port in Richtung Operationsstelle geführt wurde, schwenkt das Federelement automatisch durch die Vorspannung nach Außen, wodurch die am Federelement befestigte Vorrichtung zum Halten der chirurgischen Nadel von der gleichzeitig oder nachträglich durch das Rohr zur Operationsstelle geführten Zange der Greifeinrichtung in Abstand gehalten wird. Während des chirurgischen Nähens wird die Nadel von der Zange der Greifeinrichtung erfasst und durch das zu verbindende Gewebe gestochen und danach von der Haltevorrichtung der erfindungsgemäßen Nadelhalterkupplung übernommen. Danach wird die chirurgische Nadel wiederum von der Zange der Greifeinrichtung erfasst und erneut durch das zu verbindende Gewebe gestochen und wiederum von der Haltevorrichtung der Nadelhalterkupplung übernommen bzw. gehalten. Das elastisch nach Außen vorgespannte Federelement ist idealerweise in seitlicher Richtung nicht federnd ausgebildet und torsionsstabil, sodass die Nadelhalterkupplung beim Einstechen der Nadel nicht wesentlich verschoben wird.

In einfachster Ausführung ist die Vorrichtung zum Halten der chirurgischen Nadel durch einen Pfropfen aus elastischem Material gebildet. Dies stellt eine einfache, kostengünstige und robuste Ausführungsvariante der Haltevorrichtung dar.

Als Material für den Pfropfen in welchen die chirurgische Nadel zum vorübergehenden Halten gestochen wird eignet sich insbesondere Silikon. Dieser Kunststoff hat sich bei medizinischen Anwendungen bereits vielfach bewährt. Selbstverständlich sind aber auch andere elastische Materialien, welche entsprechende Bioverträglichkeit aufweisen, für die Herstellung des Pfropfens denkbar.

Wenn der Pfropfen lösbar mit dem Rohr der Nadelhalterkupplung verbunden ist, kann das aus sterilisierbarem Material bestehende Rohr und das Federelement der Nadelhalterkupplung wiederverwendet werden und lediglich der Pfropfen nach der Operation ausgetauscht werden. Die lösbare Verbindung des Pfropfens mit dem Rohr bzw. dem Federelement kann in einfachster Weise durch Aufstecken auf ein entsprechend gestaltetes Element erfolgen.

Die Vorrichtung zum Halten der chirurgischen Nadel kann auch durch zwei zueinander federnd vorgespannte Plättchen, zwischen welchen Plättchen die chirurgische Nadel klemmbar ist, gebildet sein. Dies stellt eine alternative Ausführungsform der Haltevorrichtung dar, die unter der Voraussetzung der Verwendung entsprechender Materialien auch sterilisierbar sein kann.

Ebenso kann die Vorrichtung zum Halten der chirurgischen Nadel durch zwei gegenüber verschiebbare Hohlzylinder gebildet sein, zwischen welchen Hohlzylindern die chirurgische Nadel klemmbar ist.

Vorteilhafterweise ist die Vorrichtung zum Halten der chirurgischen Nadel der Nadelhalterkupplung in einem seitlichen Abstand von 5 bis 20 mm von der Zange der Greifeinrichtung angeordnet. Dieser Abstand ist für die meisten Eingriffe in der Abdominalchirurgie oder Thoraxchirurgie optimal.

Wenn das Rohr und allenfalls weitere Komponenten der Nadelhalterkupplung aus sterilisierbarem Material besteht, kann das Rohr und allenfalls das daran befestigte Federelement der Nadelhalterkupplung wiederverwendet werden.

Gemäß einer weiteren Ausführungsform der Erfindung ist an der Vorrichtung zum Halten der chirurgischen Nadel ein Abstandselement angeordnet, welches durch die Greifeinrichtung seitlich auslenkbar ist. Dies stellt eine besonders einfach und kostengünstig auch aus Kunststoff herstellbare Ausführungsform der Nadelhalterkupplung dar.

Gelöst wird die Aufgabe auch durch ein oben erwähntes Instrument zum chirurgischen Nähen bei der minimal-invasiven Chirurgie, bei dem eine oben beschriebene Nadelhalterkupplung vorgesehen ist. Zu den dadurch erzielbaren Vorteilen wird auf die obige Beschreibung der Nadelhalterkupplung verwiesen.

Die Erfindung wird anhand der beigefügten Zeichnungen näher erläutert. Darin zeigen:
- Fig. 1: eine Prinzipskizze der Anwendung eines erfindungsgemäßen Instruments zum chirurgischen Nähen bei der minimal-invasiven Chirurgie;
- Fig. 2: eine Ausführungsform der Nadelhalterkupplung im Bereich des distalen Endes mit federnd vorgespanntem distalen Ende innerhalb des Ports;
- Fig. 3: die Ausführungsform der Nadelhalterkupplung gemäß Fig. 2 und der Greifeinrichtung außerhalb des Ports;
- Fig. 4: eine weitere Ausführungsform einer Nadelhalterkupplung mit zwei zueinander federnd vorgespannten Plättchen zum Klemmen einer chirurgischen Nadel;
- Fig. 5: eine weitere Ausführungsform einer Nadelhalterkupplung mit zwei gegeneinander federnd verschiebbare Hohlzylinder zum klemmen einer chirurgischen Nadel; und
- Fig. 6a: und 6b eine weitere Ausführungsform einer Nadelhalterkupplung mit einem Abstandselement mit einem das Rohr vorgeschobenen Greifeinrichtung und mit zurückgezogener Greifeinrichtung.

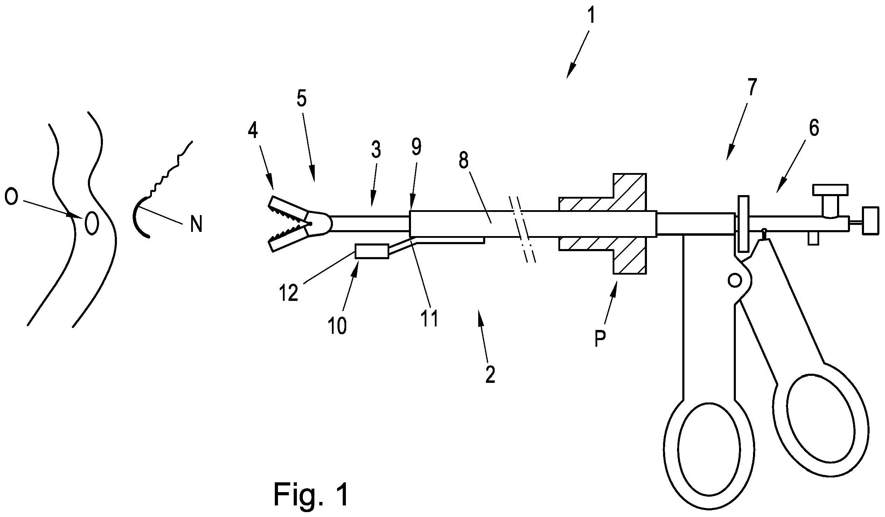

Fig. 1 zeigt eine Prinzipskizze der Anwendung eines erfindungsgemäßen Instruments 1 zum chirurgischen Nähen bei der minimal-invasiven Chirurgie, insbesondere Einzelport-Chirurgie. Dabei wird an einer Stelle über dem Operationsgebiet O, bei der Abdominalchirurgie meist im Bereich des Nabels, ein Port P gesetzt über den die Instrumente und Zuleitungen für die Zuführung des Gases zum Aufblähen der Operationsstelle O und der Zuführung einer Kamera und dergleichen (nicht dargestellt) angeordnet. Üblicherweise wird eine Greifeinrichtung 3 über eine Öffnung des Ports P an die Operationsstelle O geführt, um eine chirurgische Nadel N greifen zu können. Die Greifeinrichtung 3 weist am distalen Ende 5 eine Zange 4 zum Greifen der Nadel N und am proximalen Ende 7 ein Betätigungselement 6 zum betätigen der Zange 4 auf. Erfindungsgemäß ist eine Nadelhalterkupplung 2 vorgesehen, welche ein die Greifeinrichtung 3 umhüllendes Rohr 8 beinhaltet an dessen distalen Ende 9 eine Vorrichtung 10 zum Halten der chirurgischen Nadel N angeordnet ist. Somit ist die Vorrichtung 10 zum Halten der chirurgische Nadel N von der Zange 4 der Greifeinrichtung 3 beabstandet, wodurch das Hantieren während der Operation vereinfacht wird.

Die Nadelhalterkupplung 2 ist vorzugsweise durch ein am distalen Ende 9 des Rohres 8 angeordnetes, elastisch nach außen vorgespanntes, Federelement 11 gebildet, an welchem Federelement 11 die Vorrichtung 10 zum Halten der chirurgischen Nadel N befestigt ist. Durch die elastische Vorspannung des Federelements 11 nach Außen kann die Nadelhalterkupplung 2 durch die Öffnung des Ports P eingeführt werden. Die Vorrichtung 10 zum Halten der chirurgischen Nadel N kann durch einen Pfropfen 12 aus elastischem Material, beispielsweise Silikon, gebildet sein. In diesem Pfropfen 12 wird die chirurgische Nadel N nach dem Durchstechen des zu verbindenden Gewebes an der Operationsstelle O eingestochen und danach die Zange 4 der Greifeinrichtung 3 geöffnet und die chirurgische Nadel N wieder aus dem Pfropfen 12 aus elastischem Material gezogen und die nächste Naht an der Operationsstelle O vorgenommen. Um das Einstechen der chirurgischen Nadel N in den Pfropfen 12 zu erleichtern, sollte das nach Außen vorgespannte Federelement 11 seitlich stabil und auch torsionsstabl ausgebildet sein.

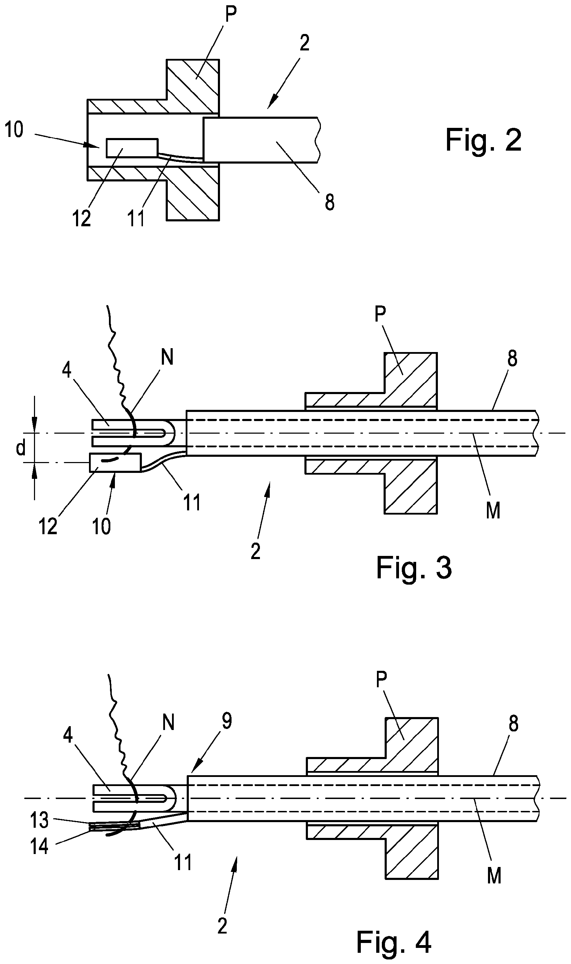

In Fig. 2 ist eine Ausführungsform der Nadelhalterkupplung 2 im Bereich des distalen Endes 9 des Rohres 8 mit federnd vorgespanntem Federelement 11 innerhalb des Ports P dargestellt. Dabei ist das Federelement 11 gegen die Federkraft nach innen gebogen, sodass die Nadelhalterkupplung 2 durch die Öffnung des Ports P passt.

Fig. 3 zeigt die Nadelhalterkupplung 2 gemäß Fig. 2 zusammen mit der Greifeinrichtung 3 in einer Lage außerhalb des Ports P. Hier schwenkt das Federelement 11 der Nadelhalterkupplung 2 durch die Federkraft automatisch nach außen, sodass ein Abstand d zwischen der Vorrichtung 10 zum Halten der chirurgischen Nadel N und der Zange 4 der Greifeinrichtung 3 gebildet wird. Der Abstand beträgt vorzugsweise 5 bis 20 mm.

In Fig. 4 ist eine weitere Ausführungsform einer Nadelhalterkupplung 2 dargestellt. Dabei wird die Vorrichtung 10 zum Halten der chirurgischen Nadel N nicht durch einen Pfropfen 12 aus elastischem Material sondern durch zwei zueinander federnd vorgespannte Plättchen 13, 14 zum Klemmen der chirurgischen Nadel N gebildet. Die zueinander federnd vorgespannten Plättchen 13, 14 können direkt am distalen Ende 9 des Rohres 8 angeordnet sein und elastisch nach außen vorgespannt sein, oder (wie dargestellt) über ein entsprechendes Federelement 11 mit dem distalen Ende 9 des Rohres 8 verbunden sein.

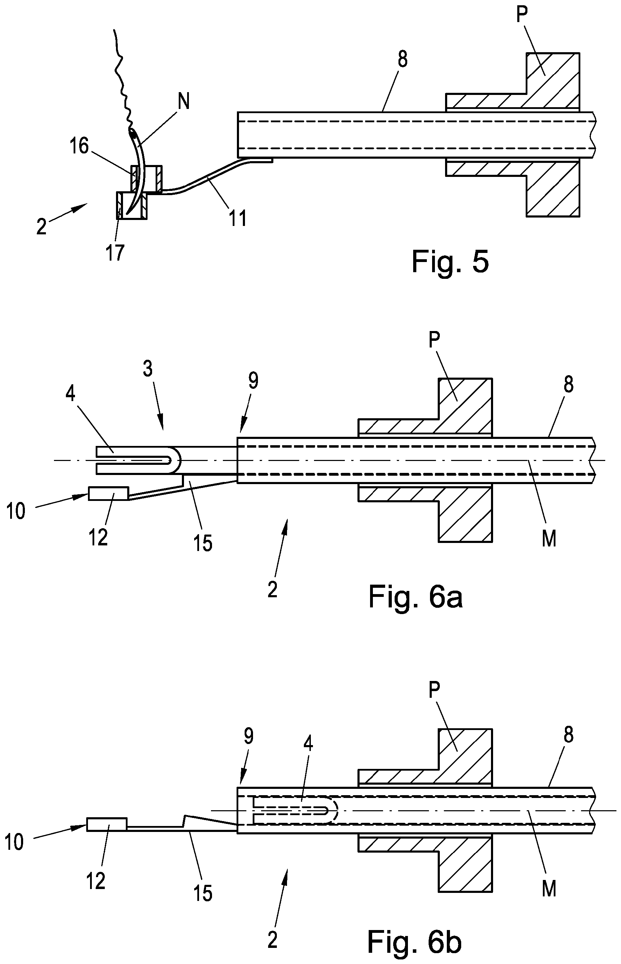

Fig. 5 zeigt eine alternative Ausführungsform einer Nadelhalterkupplung 2, wobei die Vorrichtung 10 zum Halten der chirurgischen Nadel N durch zwei zueinander verschiebbare Hohlzylinder 16, 17 gebildet wird. Die Hohlzylinder 16, 17 werden über ein entsprechendes, elastisch nach außen vorgespanntes, Federelement 11 mit dem distalen Ende 9 des Rohres 8 verbunden. Die Nadel N wird gegen einen geringen, durch eine Feder (nicht dargestellt) gebildeten Widerstand in die beiden Hohlzylinder 16, 17 eingestochen und automatisch gehalten.

Schließlich zeigen die Figuren 6a und 6b eine weitere Ausführungsform einer Nadelhalterkupplung 2, wobei die seitliche Auslenkung der Vorrichtung 10 zum Halten der chirurgischen Nadel N durch ein speziell gestaltetes Abstandselement 15 gebildet ist, welches durch die Greifeinrichtung 3 verschoben bzw. ausgelenkt werden kann. Wie in Figur 6a dargestellt, wird das keilförmig ausgebildete Abstandselement 15 durch die Greifeinrichtung 3 verschoben, sodass ein gewünschter Abstand d zwischen der Vorrichtung 10 zum Halten der chirurgischen Nadel N und der Zange 4 der Greifeinrichtung 3 gebildet wird. Figur 6b zeigt den Zustand in welchem die Greifeinrichtung 3 durch das Rohr 8 der Nadelhalterkupplung 2 zurückgezogen wird, wodurch automatisch das Abstandselement 15 mit der daran angeordneten Vorrichtung 10 zum Halten der chirurgischen Nadel N der Nadelhalterkupplung 2 in Richtung der Mittellinie M des Rohres 8 verschoben bzw. ausgelenkt wird und ohne Widerstand durch die Öffnung des Ports P entfernt werden kann. Eine derartige Ausführungsform der Nadelhalterkupplung 2 kann besonders einfach und kostengünstig auch aus Kunststoff hergestellt werden.

## Patentansprüche

1. Nadelhalterkupplung (2) für ein Instrument (1) zum chirurgischen Nähen bei der minimal-invasiven Chirurgie mit einer über einen Port (P) zu einer Operationsstelle (O) zuführbaren Greifeinrichtung (3) mit einer Zange (4) zum Greifen einer chirurgischen Nadel (N) am distalen Ende (5) und einer Einrichtung (6) zur Betätigung der Zange (4) am proximalen Ende (7), und mit einem durch den Port (P) einführbaren, die Greifeinrichtung (3) umhüllenden Rohr (8), **dadurch gekennzeichnet, dass** das Rohr (8) gegenüber der Greifeinrichtung (3) verschiebbar angeordnet ist, und am distalen Ende (9) des Rohres (8) eine gegenüber der Mittellinie (M) des Rohres (8) auslenkbare Vorrichtung (10) zum Halten der Nadel (N) angeordnet ist.

2. Nadelhalterkupplung (2) nach Anspruch 1, **dadurch gekennzeichnet, dass** am distalen Ende (9) des Rohres (8) ein elastisch nach außen vorgespanntes Federelement (11) angeordnet ist, an welchem Federelement (11) die Vorrichtung (10) zum Halten der chirurgischen Nadel (N) befestigt ist.

3. Nadelhalterkupplung (2) nach Anspruch 1 oder 2, **dadurch gekennzeichnet, dass** die Vorrichtung (10) zum Halten der chirurgischen Nadel (N) durch einen Pfropfen (12) aus elastischem Material, vorzugsweise Silikon, gebildet ist.

4. Nadelhalterkupplung (2) nach Anspruch 3, **dadurch gekennzeichnet, dass** der Pfropfen (12) lösbar mit dem Rohr (8) verbunden ist.

5. Nadelhalterkupplung (2) nach Anspruch 1 oder 2, **dadurch gekennzeichnet, dass** die Vorrichtung (10) zum Halten der Nadel (N) durch zwei zueinander federnd vorgespannte Plättchen (13, 14), zwischen welchen Plättchen (13, 14) die chirurgische Nadel (N) klemmbar ist, gebildet ist.

6. Nadelhalterkupplung (2) nach Anspruch 1 oder 2, **dadurch gekennzeichnet, dass** die Vorrichtung (10) zum Halten der chirurgischen Nadel (N) durch zwei gegeneinander federnd verschiebbare Hohlzylinder (16, 17), zwischen welchen Hohlzylindern (16, 17) die chirurgische Nadel (N) klemmbar ist, gebildet ist.

7. Nadelhalterkupplung (2) nach einem der Ansprüche 1 bis 6, **dadurch gekennzeichnet, dass** die Vorrichtung (10) zum Halten der chirurgischen Nadel (N) in einem seitlichen Abstand (d) von 5 bis 20 mm von der Mittellinie (M) des Rohres (8) angeordnet ist.

8. Nadelhalterkupplung (2) nach einem der Ansprüche 1 bis 7, **dadurch gekennzeichnet, dass** das Rohr (8) aus sterilisierbarem Material besteht.

9. Nadelhalterkupplung (2) nach einem der Ansprüche 1 bis 8, **dadurch gekennzeichnet, dass** an der Vorrichtung (10) zum Halten der chirurgischen Nadel (N) ein Abstandselement (15) angeordnet ist, welches durch die Greifeinrichtung (3) seitlich auslenkbar ist.

10. Instrument (1) zum chirurgischen Nähen bei der minimal-invasiven Chirurgie, mit einer über einen Port (P) zu einer Operationsstelle (O) zuführbaren Greifeinrichtung (3) mit einer Zange (4) zum Greifen einer chirurgischen Nadel (N) am distalen Ende (5) und einer Einrichtung (6) zur Betätigung der Zange (4) am proximalen Ende (7), **dadurch gekennzeichnet, dass** eine durch den Port (P) einführbare Nadelhalterkupplung (2) nach einem der Ansprüche 1 bis 9 vorgesehen ist.

## Claims

1. A needle holder coupling (2) for an instrument (1) for performing surgical sutures in minimally invasive surgery, comprising gripper means (3) to be supplied to a surgical site (O) via a port (P), said gripper means (3) comprising tongs (4) for gripping a surgical needle (N) at the distal end (5) and means (6) for actuating the tongs (4) at the proximal end (7), and comprising a tube (8) adapted to be inserted through the port (P) and surrounding the gripper means (3), **characterized in that** the tube (8) is arranged to be displaced in relation to the gripper means (3) and that, at the distal end (9) of the tube (8), a device (10) deflectable in relation to the center line (M) of the tube (8) is arranged for holding the needle (N).

2. The needle holder coupling (2) according to claim 1, **characterized in that**, at the distal end (9) of the tube (8), a spring element (11) biased elastically outwardly is arranged, at which spring element (11) the device (10) for holding the surgical needle (N) is fastened.

3. The needle holder coupling (2) according to claim 1 or 2, **characterized in that** the device (10) for holding the surgical needle (N) is formed by a plug (12) of elastic material, preferably silicone.

4. The needle holder coupling (2) according to claim 3, **characterized in that** the plug (12) is releasably connected with the tube (8).

5. The needle holder coupling (2) according to claim 1 or 2, **characterized in that** the device (10) for holding the needle (N) is formed by two small plates (13, 14) biased resiliently with respect to each other, between which small plates (13, 14) the surgical needle (N) can be clamped.

6. The needle holder coupling (2) according to claim 1 or 2, **characterized in that** the device (10) for holding the surgical needle (N) is formed by two hollow cylinders (16, 17) adapted to be displaced resiliently against each other, between which hollow cylinders (16, 17) the surgical needle (N) can be clamped.

7. The needle holder coupling (2) according to any of claims 1 to 6, **characterized in that** the device (10) for holding the surgical needle (N) is arranged at a lateral distance (d) of 5 to 20 mm from the center line (M) of the tube (8).

8. The needle holder coupling (2) according to any of claims 1 to 7, **characterized in that** the tube (8) consists of sterilizable material.

9. The needle holder coupling (2) according to any of claims 1 to 8, **characterized in that** a spacer element (15) is arranged at the device (10) for holding the surgical needle (N), said spacer element (15) being laterally deflectable by the gripper means (3).

10. An instrument (1) for performing a surgical suture in minimally invasive surgery, comprising gripper means (3) to be supplied to a surgical site (O) via a port (P), said gripper means (3) comprising tongs (4) for gripping a surgical needle (N) at the distal end (5) and means (6) for actuating the tongs (4) at the proximal end (7), **characterized in that** a needle holder coupling (2) insertable through the port (P) in accordance with any of claims 1 to 9 is provided.

## Revendications

1. Couplage de porte-aiguille (2) pour un instrument (1) pour la suture chirurgicale lors de la chirurgie mini-invasive avec un dispositif de préhension (3) pouvant être amené par un orifice (P) jusqu'à un site opératoire (O), avec une pince (4) pour saisir une aiguille (N) chirurgicale à l'extrémité distale (5) et un dispositif (6) pour actionner la pince (4) à l'extrémité proximale (7), et avec un tube (8) pouvant être inséré dans l'orifice (P), entourant le dispositif de préhension (3), **caractérisé en ce que** le tube (8) est disposé de façon à pouvoir coulisser par rapport au dispositif de préhension (3) et à l'extrémité distale (9) du tube (8) est disposé un système (10) pouvant être dévié par rapport à la ligne médiane (M) du tube (8) pour le maintien de l'aiguille (N).

2. Couplage de porte-aiguille (2) selon la revendication 1, **caractérisé en ce qu'**à l'extrémité distale (9) du tube (8) est disposé un élément à ressort (11) précontraint élastiquement vers l'extérieur, auquel élément à ressort (11) le système (10) pour le maintien de l'aiguille (N) chirurgicale est fixé.

3. Couplage de porte-aiguille (2) selon la revendication 1 ou 2, **caractérisé en ce que** le système (10) de maintien de l'aiguille (N) chirurgicale est formé par un tampon (12) d'un matériau élastique, de préférence de silicone.

4. Couplage de porte-aiguille (2) selon la revendication 3, **caractérisé en ce que** le tampon (12) est relié au tube (8) de façon amovible.

5. Couplage de porte-aiguille (2) selon la revendication 1 ou 2, **caractérisé en ce que** le dispositif (10) de maintien de l'aiguille (N) est formé par deux plaquettes (13, 14) précontraintes élastiquement l'une par rapport à l'autre, entre lesquelles plaquettes (13, 14) l'aiguille (N) chirurgicale peut être serrée.

6. Couplage de porte-aiguille (2) selon la revendication 1 ou 2, **caractérisé en ce que** le système (10) de maintien de l'aiguille (N) chirurgicale est formé par deux cylindres creux (16, 17) pouvant être décalés élastiquement l'un par rapport à l'autre, entre lesquels cylindres creux (16, 17) l'aiguille (N) chirurgicale peut être serrée.

7. Couplage de porte-aiguille (2) selon l'une des revendications 1 à 6, **caractérisé en ce que** le système (10) de maintien de l'aiguille (N) chirurgicale est disposé à une distance latérale (d) de 5 à 20 mm de la ligne médiane (M) du tube (8).

8. Couplage de porte-aiguille (2) selon l'une des revendications 1 à 7, **caractérisé en ce que** le tube (8) est constitué d'un matériau stérilisable.

9. Couplage de porte-aiguille (2) selon l'une des revendications 1 à 8, **caractérisé en ce que** sur le système (10) de maintien de l'aiguille (N) chirurgicale est disposé un élément d'espacement (15) qui peut être dévié latéralement par le dispositif de préhension (3).

10. Instrument (1) pour la suture chirurgicale lors de la chirurgie mini-invasive, avec un dispositif de préhension (3) pouvant être amené par un orifice (P) jusqu'à un site opératoire (O), avec une pince (4) pour saisir une aiguille (N) chirurgicale à l'extrémité distale (5) et un dispositif (6) pour actionner la pince (4) à l'extrémité proximale (7), **caractérisé en ce qu'**un couplage de porte-aiguille (2) pouvant être inséré dans l'orifice (P) est prévu selon l'une des revendications 1 à 9.
